# EUROPEAN PATENT SPECIFICATION

(11) **EP 4 435 922 B1**
(45) Date of publication and mention of the grant of the patent: **23.04.2025**
(21) Application number: 23209074.6
(22) Date of filing: 10.11.2023
(51) Int. Cl.: H01M 10/0569, H01M 10/0567, H01M 10/0525, H01M 4/525, H01M 10/42, H01M 4/36, H01M 4/38, H01M 4/587, H01M 4/62

(54) **RECHARGEABLE LITHIUM BATTERY**
WIEDERAUFLADBARE LITHIUMBATTERIE
BATTERIE RECHARGEABLE AU LITHIUM

(30) Priority: 23.03.2023 KR 20230038064
(43) Date of publication of application: 25.09.2024
(73) Proprietor: Samsung SDI Co., Ltd., Yongin-si, Gyeonggi-do 17084 (KR)
(72) Inventor: Park, Sangwoo, 17084 Yongin-si, Gyeonggi-do (KR); Kim, Sanghoon, 17084 Yongin-si, Gyeonggi-do (KR); Choi, Hyunbong, 17084 Yongin-si, Gyeonggi-do (KR); Kim, Sundae, 17084 Yongin-si, Gyeonggi-do (KR); Kim, Dahyun, 17084 Yongin-si, Gyeonggi-do (KR); Park, Hongryeol, 17084 Yongin-si, Gyeonggi-do (KR); Yang, Yeji, 17084 Yongin-si, Gyeonggi-do (KR)
(74) Representative: Gulde & Partner

(56) References cited:
- EP-A1- 3 806 223
- EP-A1- 4 040 527
- KR-A- 20190 017 477
- US-A1- 2014 186 722
- US-A1- 2019 089 003
- X. ZHENG ET AL: "Exploring high-voltage fluorinated carbonate electrolytes for LiNi0.5Mn1.5O4 cathode in Li-ion batteries", JOURNAL OF ENERGY CHEMISTRY, vol. 42, 4 June 2019 (2019-06-04), pages 62 - 70, XP085914418, ISSN: 2095-4956, [retrieved on 20190604], DOI: 10.1016/J.JECHEM.2019.05.023

## Description

### BACKGROUND OF THE INVENTION.

### 1. Field

Embodiments of the present disclosure described herein are related to a rechargeable lithium battery.

### 2. Description of Related Art

A rechargeable lithium battery may be recharged and has three or more times as high (e.g., at least three-times) the energy density per unit weight as a comparable lead storage battery, nickel-cadmium battery, nickel hydrogen battery, nickel zinc battery and/or the like. Rechargeable lithium batteries may be also charged at a relatively high rate and thus, are commercially manufactured for a laptop, a cell phone, an electric tool, an electric bike, and/or the like, and improvements of additional energy density have been sought.

In particular, as IT (informational technology) devices increasingly achieve high performance, a high-capacity battery is required or desired, but the relatively high capacity may be realized through expansion of a voltage range, which may increase energy density but may also bring a problem of deteriorating performance of a positive electrode due to oxidization of an electrolyte in the high voltage range.

In particular, a cobalt-free lithium nickel manganese-based oxide is a positive electrode active material including not cobalt but nickel, manganese, and/or the like as a main component in its composition, and accordingly, a positive electrode including the same may be economical and realize relatively high energy density and thus draws attention as a next generation positive electrode active material.

However, when the positive electrode including the cobalt-free lithium nickel manganese-based oxide is utilized in a high voltage environment, transition metals may be eluted due to structural collapse of the positive electrode, thereby causing a problem such as gas generation inside a cell, capacity reduction, and/or the like. This transition metal elution tends to be aggravated in a high temperature environment, wherein the eluted transition metals are precipitated on the surface of a negative electrode and may cause a side reaction and thereby increase battery resistance and deteriorate battery cycle-life and output characteristics.

Accordingly, when the positive electrode including the cobalt-free lithium nickel manganese-based oxide is utilized, an electrolyte applicable under high voltage and high temperature conditions is desired or required.

X. Zheng, et al., J. Energy Chem. 2020, 42, 62-70, discloses high-voltage fluorinated carbonate electrolytes for a lithium nickel manganese-based oxide cathode in lithium-ion batteries. US 2019/0089003 A1 describes an electrolyte solution for secondary lithium-ion batteries, comprising an organic solvent mixture including at least one sulfone compound, at least one fluorine-containing cyclic acetal compound and a cyclic carbonate compound. Moreover, KR 2019 0017477 A reveals a non-aqueous electrolytic solution for secondary lithium-ion batteries, comprising a lithium salt and a surfactant. In EP 3 806 223 A1 an electrolyte solution, comprising a carbonate and a high-oxidation-potential solvent, is described. Beyond that, US 2014/186722 A1 discloses a non-aqueous electrolyte solution for a secondary lithium-ion battery including a non-aqueous organic solvent including propylene carbonate and lithium bis(fluorosulfonyl)imide. Finally, EP 4 040 527 A1 reveals a non-aqueous electrolyte for a secondary lithium-ion battery system comprising a lithium transition metal composite oxide containing 85mol% or more of Ni and 1mol% or more and 15mol% or less of Al.

### SUMMARY

The invention is defined by the appended claims. The description that follows is subjected to this limitation. Any disclosure lying outside the scope of said claims is only intended for illustrative as well as comparative purposes.

An aspect according to one or more embodiments is directed toward a rechargeable lithium battery exhibiting improved high-voltage characteristics and high-temperature characteristics by combining a positive electrode including lithium nickel manganese-based oxide with an electrolyte capable of effectively protecting the positive electrode including a lithium nickel manganese-based oxide to reduce elution of transition metals under high-voltage and high-temperature conditions and thus to suppress or reduce structural collapse of the positive electrode.

Additional aspects will be set forth in part in the description which follows and, in part, will be apparent from the description, or may be learned by practice of the presented embodiments of the disclosure.

According to the present invention, a rechargeable lithium battery includes an electrolyte including a non-aqueous organic solvent, a lithium salt, and an additive; a positive electrode including a positive electrode active material; and a negative electrode including a negative electrode active material,

wherein the non-aqueous organic solvent is only composed of a chain carbonate,

the positive electrode active material includes a cobalt-free lithium nickel manganese-based oxide, wherein the positive electrode active material is represented by Chemical Formula 4:

Chemical Formula 4 LiₐNiₓMn_{y}M¹_{z}M²_{w}O_{2±b}X_{c}, and

wherein, in Chemical Formula 4, 0.5 ≤ a < 1.8, 0 ≤ b ≤ 0.1, 0 ≤ c ≤ 0.1, 0 ≤ w < 0.1, 0.6 ≤ x < 1.0, 0<y<0.4, 0 < z < 0.1, w + x + y + z = 1, M¹ and M² are each independently one or more elements selected from among Al, Mg, Ti, Zr, Sr, V, B, W, Mo, Si, Ba, Ca, Ce, Cr, Fe, and Nb, and X is one or more elements selected from among S, F, P, and Cl, and
the additive includes a compound represented by Chemical Formula 1:

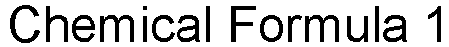

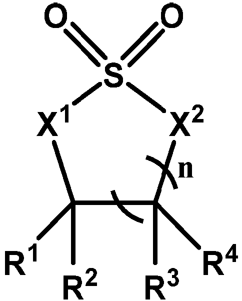

In Chemical Formula 1,
X¹ and X² are each independently be O, S, or CR^{a}R^{b},
at least one selected from among X¹ and X² is O,
R^{a}, R^{b}, and R¹ to R⁴ are each independently be hydrogen, a halogen, a substituted or unsubstituted C1 to C20 alkyl group, a substituted or unsubstituted C1 to C20 alkoxy group, a substituted or unsubstituted C2 to C20 alkenyl group, a substituted or unsubstituted C3 to C20 alkynyl group, a substituted or unsubstituted C3 to C20 cycloalkyl group, a substituted or unsubstituted C6 to C50 aryl group, a substituted or unsubstituted C7 to C50 alkylaryl group, or a substituted or unsubstituted C6 to C50 heteroaryl group, and
n is an integer of 1 or 2.

The term "substituted" refers to replacement of at least one selected from among hydrogen of a substituent or a compound by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

Some embodiments may realize a rechargeable lithium battery exhibiting improved battery stability and cycle-life characteristics by combining a positive electrode including a lithium nickel manganese-based oxide with an electrolyte capable of effectively protecting the positive electrode to secure phase transition safety of the positive electrode in a high temperature high voltage environment and to suppress or reduce decomposition of the electrolyte and a side reaction with electrodes and thus reduce gas generation and concurrently (e.g., simultaneously), suppress or reduce an increase in battery internal resistance.

### BRIEF DESCRIPTION OF THE DRAWINGS

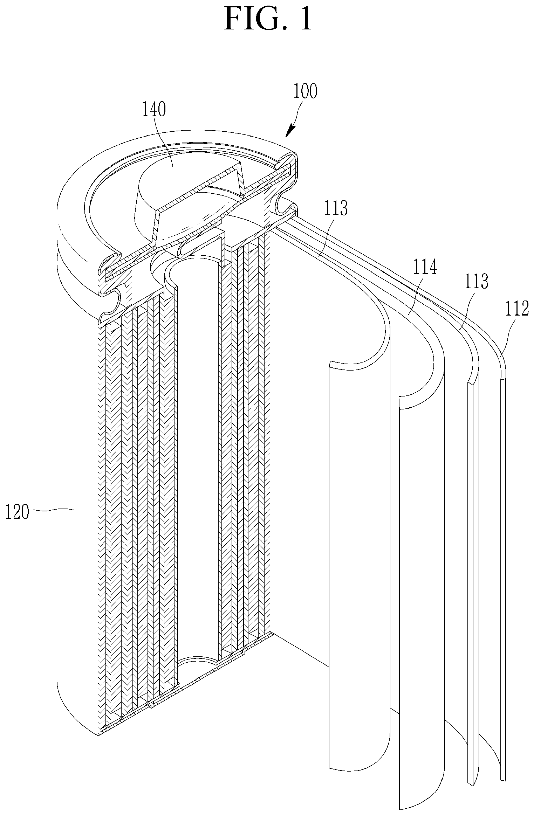

The drawing is a schematic view illustrating a rechargeable lithium battery according to some embodiments.

### DETAILED DESCRIPTION

Hereinafter, a rechargeable lithium battery according to some embodiments of the present disclosure will be described in more detail with reference to the accompanying drawings.

In the present specification, when a definition is not otherwise provided, "substituted" refers to replacement of at least one selected from among hydrogen of a substituent or a compound by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

In one example of the present disclosure, "substituted" refers to replacement of at least one selected from among hydrogen of a substituent or a compound by deuterium, a halogen, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C10 fluoroalkyl group, and a cyano group. In some embodiments, in specific examples of the present disclosure, "substituted" refers to replacement of at least one selected from among hydrogen of a substituent or a compound by deuterium, a halogen, a C1 to C20 alkyl group, a C6 to C30 aryl group, a C1 to C10 fluoroalkyl group, and a cyano group. In some embodiments, in specific examples of the present disclosure, "substituted" refers to replacement of at least one selected from among hydrogen of a substituent or a compound by deuterium, a halogen, a C1 to C5 alkyl group, a C6 to C18 aryl group, a C1 to C5 fluoroalkyl group, and a cyano group. In some embodiments, in specific examples of the present disclosure, "substituted" refers to replacement of at least one selected from among hydrogen of a substituent or a compound by deuterium, a cyano group, a halogen, a methyl group, an ethyl group, a propyl group, a butyl group, a phenyl group, a biphenyl group, a terphenyl group, a trifluoromethyl group, and a naphthyl group.

A rechargeable lithium battery may be classified into a lithium ion battery, a lithium ion polymer battery, and a lithium polymer battery depending on types (kinds) of a separator and an electrolyte and also may be classified to be cylindrical, prismatic, coin-type or kind, pouch-type or kind, and/or the like depending on shapes. In some embodiments, it may be bulk type or kind and thin film type or kind depending on sizes. Structures and manufacturing methods for rechargeable lithium batteries pertaining to this disclosure are suitable in the art.

Herein, a cylindrical rechargeable lithium battery will be described as an example of the rechargeable lithium battery. The drawing schematically shows the structure of a rechargeable lithium battery according to some embodiments. Referring to the drawing, a rechargeable lithium battery 100 includes a battery cell including a positive electrode 114, a negative electrode 112 facing the positive electrode 114, a separator 113 between the positive electrode 114 and the negative electrode 112, and an electrolyte impregnating the positive electrode 114, the negative electrode 112, and the separator 113, a battery case 120 housing the battery cell, and a sealing member 140 sealing the battery case 120.

Hereinafter, a more detailed configuration of the rechargeable lithium battery 100 according to some embodiments of the present disclosure will be described.

A rechargeable lithium battery according to the invention includes an electrolyte, a positive electrode, and a negative electrode.

The electrolyte includes a non-aqueous organic solvent, a lithium salt, and an additive, the non-aqueous organic solvent is only composed of chain carbonate, and the additive includes a compound represented by Chemical Formula 1.

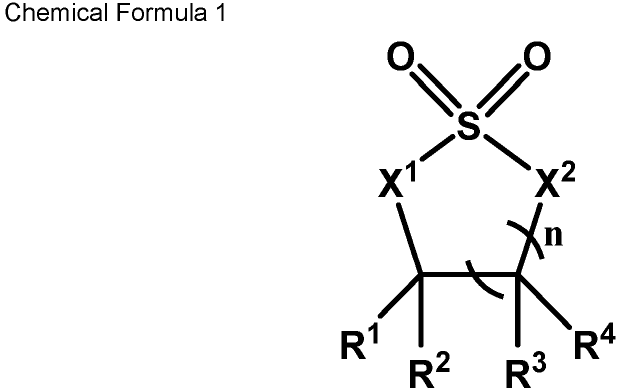

In Chemical Formula 1,
X¹ and X² are each independently be O, S, or CR^{a}R^{b},
at least one selected from among X¹ and X² is O,
R^{a}, R^{b}, and R¹ to R⁴ are each independently be hydrogen, a halogen, a substituted or unsubstituted C1 to C20 alkyl group, a substituted or unsubstituted C1 to C20 alkoxy group, a substituted or unsubstituted C2 to C20 alkenyl group, a substituted or unsubstituted C3 to C20 alkynyl group, a substituted or unsubstituted C3 to C20 cycloalkyl group, a substituted or unsubstituted C6 to C50 aryl group, a substituted or unsubstituted C7 to C50 alkylaryl group, or a substituted or unsubstituted C6 to C50 heteroaryl group, and
n is an integer of 1 or 2.

The positive electrode includes a positive electrode active material including a cobalt-free lithium nickel manganese-based oxide.

In the case of a positive electrode active material including lithium nickel manganese-based oxide, structural instability is severe under high-voltage conditions, resulting in solvent decomposition and elution of transition metals, particularly Ni.

Due to such a transition metal elution phenomenon, deterioration of performance of a positive electrode and short-circuiting caused by elution of transition metals occur, resulting in a decrease in cycle-life characteristics of the battery and a rapid increase in resistance.

However, in the case of utilizing the aforementioned electrolyte together, it is possible to alleviate a decrease in the cycle-life characteristics of the battery and the rapid increase in resistance.

In particular, by utilizing a positive electrode including a lithium nickel manganese-based oxide in an electrolyte, which is free of ethylene carbonate (except for indistinguishable traces left by the synthesis of other non-aqueous organic solvents), the elution of transition metals can be effectively reduced under high-voltage and high-temperature conditions. Accordingly, the high-voltage characteristics and high-temperature characteristics of the battery can be improved by suppressing the collapse of the positive electrode structure.

The non-aqueous organic solvent serves as a medium for transmitting ions taking part in the electrochemical reaction of a battery.

The non-aqueous organic solvent may be a carbonate-based, ester-based, ether-based, ketone-based, alcohol-based, or aprotic solvent.

The carbonate-based solvent may include at least one of dimethyl carbonate (DMC), diethyl carbonate (DEC), dipropyl carbonate (DPC), methylpropyl carbonate (MPC), ethylpropyl carbonate (EPC), ethylmethyl carbonate (EMC), ethylene carbonate (EC), propylene carbonate (PC), and butylene carbonate (BC). The ester-based solvent may include at least one of methyl acetate, ethyl acetate, n-propyl acetate, t-butyl acetate, methylpropionate, ethylpropionate, propylpropionate, decanolide, mevalonolactone, and caprolactone. The ether-based solvent may include at least one of dibutyl ether, tetraglyme, diglyme, dimethoxyethane, 2-methyltetrahydrofuran, and tetrahydrofuran. In some embodiments, the ketone-based solvent may include cyclohexanone. The alcohol-based solvent may include at least one of ethanol, and isopropyl alcohol and the aprotic solvent may include nitriles such as R-CN (wherein R is a hydrocarbon group having a C2 to C20 linear, branched, or cyclic structure and may include a double bond, an aromatic ring, or an ether bond), amides such as dimethyl formamide, dioxolanes such as 1,3-dioxolane, or sulfolanes.

The non-aqueous organic solvent may be utilized alone or in a mixture, and when the non-aqueous organic solvent is utilized in a mixture, the mixture ratio may be controlled or selected in accordance with a desirable battery performance.

The non-aqueous organic solvent includes less than 5 wt% of ethylene carbonate (which is a cyclic carbonate ester) based on the total weight of the non-aqueous organic solvent.

When the content (e.g., amount) of ethylene carbonate is included in an amount greater than or equal to 5 wt%, an activity of Ni increases during high-voltage operation, and an oxidation number of Ni tends to be reduced from tetravalent to divalent, and ethylene carbonate, which has low oxidation stability, is oxidatively decomposed, and as a result, Ni is eluted and deposited on the negative electrode.

The non-aqueous organic solvent is composed of only chain carbonate. A "chain carbonate" is compound including a carbonate group and two substituted or non-substituted alkyl moieties bond to the carbonate group, but not forming a cycle together with the carbonate group of the compound. In this case, as the resistance increase rate during high-temperature storage is significantly alleviated, excellent or suitable high-temperature storage characteristics may be implemented.

As utilized herein, "composed of only chain carbonate" refers to including non-aqueous organic solvents belonging to the category of chain carbonates alone or in combination without being mixed with cyclic carbonates.

In some embodiments, the chain carbonate may be represented by Chemical Formula 2.

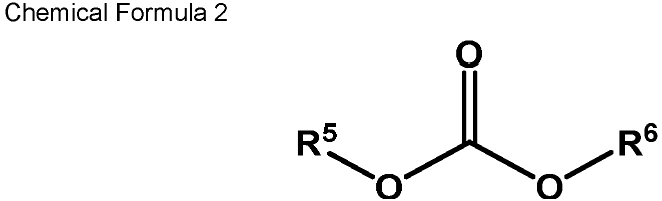

In Chemical Formula 2
R⁵ and R⁶ are each independently be a substituted or unsubstituted C1 to C20 alkyl group.

For example, R⁵ and R⁶ in Chemical Formula 2 may each independently be a substituted or unsubstituted C1 to C10 alkyl group, and for example the R⁵ and R⁶ may each independently be a substituted or unsubstituted C1 to C5 alkyl group.

In some embodiments, R⁵ and R⁶ in Chemical Formula 2 may each independently be a substituted or unsubstituted methyl group, a substituted or unsubstituted ethyl group, a substituted or unsubstituted n-propyl group, a substituted or unsubstituted n-butyl group, a substituted or unsubstituted n-pentyl group, a substituted or unsubstituted iso-butyl group, or a substituted or unsubstituted neopentyl group.

For example, the non-aqueous organic solvent according to some embodiments may be a mixed solvent of at least two solvents selected from among dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

The non-aqueous organic solvent according to some embodiments may be dimethyl carbonate (DMC) or a combination of dimethyl carbonate (DMC) and ethylmethyl carbonate (EMC).

The non-aqueous organic solvent may include ethylmethyl carbonate (EMC) and dimethyl carbonate (DMC) in a volume ratio of about 0:100 to about 50:50.

It may be more desirable in terms of improving battery characteristics that the non-aqueous organic solvent includes dimethyl carbonate (DMC) in an amount of more than about 50 volume%.

For example, the non-aqueous organic solvent may include ethylmethyl carbonate (EMC) and dimethyl carbonate (DMC) in a volume ratio of about 0:100 to about 40:60, about 0:100 to about 30:70, about 10:90 to about 40:60, or about 10:90 to about 30:70.

The non-aqueous organic solvent may further include an aromatic hydrocarbon-based organic solvent in addition to the chain carbonate-based solvent. Herein, the chain carbonate-based solvent and the aromatic hydrocarbon-based organic solvent may be mixed in a volume ratio of about 1:1 to about 30:1.

The aromatic hydrocarbon-based organic solvent may be an aromatic hydrocarbon-based compound represented by Chemical Formula 3.

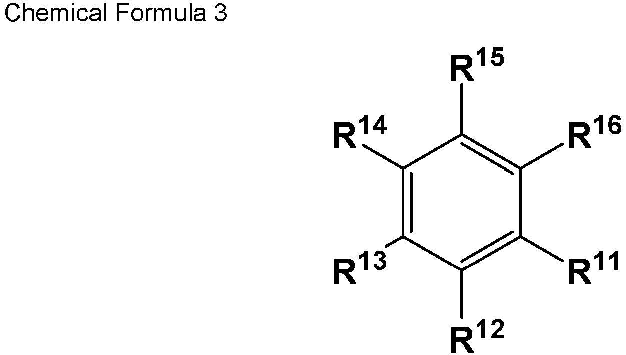

In Chemical Formula 3, R¹¹ to R¹⁶ may each independently be the same or different and are selected from among hydrogen, a halogen, a C1 to C10 alkyl group, a C1 to C10 haloalkyl group, and a combination thereof.

Examples of the aromatic hydrocarbon-based organic solvent may be selected from among benzene, fluorobenzene, 1,2-difluorobenzene, 1,3-difluorobenzene, 1,4-difluorobenzene, 1,2,3-trifluorobenzene, 1,2,4-trifluorobenzene, chlorobenzene, 1,2-dichlorobenzene, 1,3-dichlorobenzene, 1,4-dichlorobenzene, 1,2,3-trichlorobenzene, 1,2,4-trichlorobenzene, iodobenzene, 1,2-diiodobenzene, 1,3-diiodobenzene, 1,4-diiodobenzene, 1,2,3-triiodobenzene, 1,2,4-triiodobenzene, toluene, fluorotoluene, 2,3-difluorotoluene, 2,4-difluorotoluene, 2,5-difluorotoluene, 2,3,4-trifluorotoluene, 2,3,5-trifluorotoluene, chlorotoluene, 2,3-dichlorotoluene, 2,4-dichlorotoluene, 2,5-dichlorotoluene, 2,3,4-trichlorotoluene, 2,3,5-trichlorotoluene, iodotoluene, 2,3-diiodotoluene, 2,4-diiodotoluene, 2,5-diiodotoluene, 2,3,4-triiodotoluene, 2,3,5-triiodotoluene, xylene, and a combination thereof.

The lithium salt dissolved in the non-aqueous organic solvent supplies lithium ions in a battery, enables a basic operation of a rechargeable lithium battery, and improves transportation of the lithium ions between positive and negative electrodes. Examples of the lithium salt include at least one supporting salt selected from among LiPF₆, LiBF₄, lithium difluoro(oxalate)borate (LiDFOB), LiPO₂F₂, LiSbF₆, LiAsF₆, Li(FSO₂)₂N (lithium bis(fluorosulfonyl)imide (LiFSI), LiC₄F₉SO₃, LiClO₄, LiAlO₂, LiAICl₄, LiN(CₓF₂ₓ₊₁ SO₂)(C_{y}F_{2y+1} SO₂), wherein, x and y may each independently be an integer selected from 1 to 20, LiCl, Lil, and LiB(C₂O₄)₂ (lithium bis(oxalato) borate: LiBOB).

The lithium salt may be utilized in a concentration in a range of about 0.1 M to about 2.0 M. When the lithium salt is included at the above concentration range, an electrolyte may have excellent or suitable performance and lithium ion mobility due to optimal or suitable electrolyte conductivity and viscosity.

Chemical Formula 1 may be represented by Chemical Formula 1A or Chemical Formula 1B.

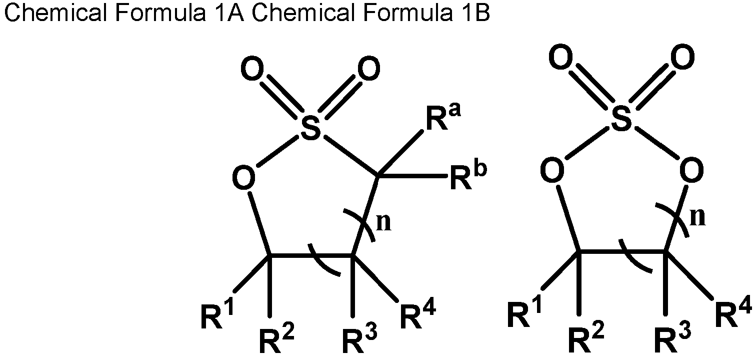

In Chemical Formula 1A and Chemical Formula 1B,
R^{a}, R^{b}, R¹ to R⁴, and n are each independently be the same as described above.

For example, R^{a}, R^{b}, and R¹ to R⁴ in Chemical Formula 1 may each independently be hydrogen, a substituted or unsubstituted C1 to C10 alkyl group, or a substituted or unsubstituted C6 to C20 aryl group.

For example, R^{a}, R^{b}, and R¹ to R⁴ in Chemical Formula 1 may each independently be hydrogen or a substituted or unsubstituted C1 to C6 alkyl group.

For example, R^{a}, R^{b}, and R¹ to R⁴ in Chemical Formula 1 may each independently be hydrogen or a substituted or unsubstituted C1 to C3 alkyl group.

For example, n in Chemical Formula 1 may be 1.

The compound represented by Chemical Formula 1 may be selected from among the compounds listed in Group 1.
Group 1 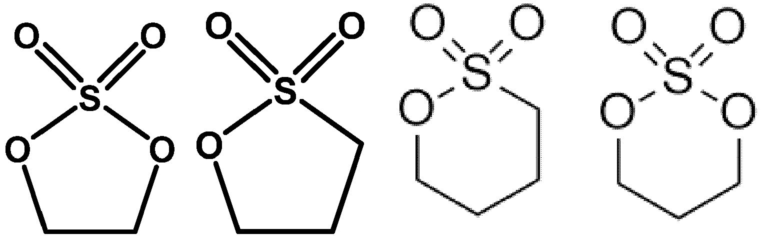 The compound represented by Chemical Formula 1 may be included in an amount of 0.05 to 5.0 parts by weight based on 100 parts by weight of the total electrolyte excluding additives (i.e. based on the total weight of lithium salt and non-aqueous organic solvent).

For example, the compound represented by Chemical Formula 1 may be included in an amount of 0.05 to 3.0 parts by weight based on 100 parts by weight of the total electrolyte excluding additives.

For example, the compound represented by Chemical Formula 1 may be included in an amount of 0.1 to 3.0 parts by weight, about 0.3 to 3.0 parts by weight, 0.5 to 3.0 parts by weight, or 0.5 to 2.0 parts by weight based on 100 parts by weight of the total electrolyte excluding additives.

When the content (e.g., amount) range of the compound represented by Chemical Formula 1 is as described above, an increase in resistance at high temperatures may be prevented or reduced, and thus a rechargeable lithium battery having improved cycle-life characteristics and output characteristics may be implemented.

The electrolyte may further include at least one selected from among other additives of vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene Carbonate, vinylethylene carbonate (VEC), adiponitrile (AN), succinonitrile (SN), 1,3,6-hexane tricyanide (HTCN), propene sultone (PST), propane sultone (PS), lithium tetrafluoroborate (LiBF₄), lithium difluorophosphate (LiPO₂F₂), and 2-fluoro biphenyl (2-FBP).

By further including the aforementioned other additives, cycle-life may be further improved or gases generated from the positive electrode and the negative electrode may be effectively controlled or selected during high-temperature storage.

The other additives may be included in an amount of about 0.2 to about 20 parts by weight, specifically about 0.2 to about 15 parts by weight, for example about 0.2 to about 10 parts by weight based on the total weight of the electrolyte for a rechargeable lithium battery.

When the content (e.g., amount) of other additives is as described above, the increase in film resistance may be minimized or reduced, thereby contributing to the improvement of battery performance.

The positive electrode includes a positive electrode current collector and a positive electrode active material layer on the positive electrode current collector, and the positive electrode active material layer includes a positive electrode active material.

The positive electrode active material includes a cobalt-free lithium nickel manganese-based oxide.

The lithium nickel manganese-based oxide includes at least one of cobalt-free lithium composite oxides represented by Chemical Formula 4.

Chemical Formula 4 LiₐNiₓMn_{y}M¹_{z}M²_{w}O_{2±b}X_{c}

In Chemical Formula 4,
0.5 ≤ a < 1.8, 0 ≤ b ≤ 0.1, 0 ≤ c ≤ 0.1, 0 ≤ w < 0.1, 0.6 ≤ x < 1.0, 0 < y < 0.4, 0 < z < 0.1, w + x + y + z = 1,
M¹ and M² are each independently one or more elements selected from among Al, Mg, Ti, Zr, Sr, V, B, W, Mo, Si, Ba, Ca, Ce, Cr, Fe, and Nb, and X is one or more elements selected from S, F, P, and Cl.

As utilized herein, cobalt-free lithium composite oxide as a positive electrode active material refers to a positive electrode active material including nickel, manganese, etc. as a main component without including cobalt in the composition of the positive electrode active material.

The lithium composite oxide may have a coating layer on the surface, or may be mixed with another compound having a coating layer. The coating layer may include at least one coating element compound selected from among an oxide of a coating element, a hydroxide of a coating element, an oxyhydroxide of a coating element, an oxycarbonate of a coating element, and a hydroxy carbonate of a coating element. The compound for the coating layer may be amorphous or crystalline. The coating element included in the coating layer may include Mg, Al, K, Na, Ca, Si, Ti, V, Sn, Ge, Ga, B, As, Zr, or a mixture thereof. The coating process may include any suitable process(es) as long as they do not cause any or any substantial side effects on the properties of the positive electrode active material (e.g., spray coating, dipping), which should be apparent to persons having ordinary skill in this art, so a detailed description thereof is not provided.

For example, Chemical Formula 4 may be represented by Chemical Formula 4-1.

Chemical Formula 4-1 LiₐNiₓ₁Mn_{y1}Al_{z1}M²_{w1}O_{2±b}X_{c}

In Chemical Formula 4-1,
0.5 ≤ a < 1.8, 0 ≤ b ≤ 0.1, 0 ≤ c ≤ 0.1, 0 ≤ w1 < 0.1, 0.6 ≤ x1 < 1.0, 0 < y1 < 0.4, 0 < z1 < 0.1, w1 + x1 + y1 + z1 = 1,
M² is one or more elements selected from among Mg, Ti, Zr, Cr, Sr, V, B, W, Mo, Si, Ba, Ca, Ce, Fe, and Nb, and X is one or more elements selected from among S, F, P, and Cl.

In some embodiments, in Chemical Formula 4-1, 0.6≤x1≤0.9, 0.1≤y1< 0.4, and 0 < z1 < 0.1, or 0.6≤x1≤0.8, 0.2≤y1 < 0.4, and 0 < z1 < 0.1.

For example, in Chemical Formula 4-1, x1 may be 0.6 ≤ x1 ≤0.79, y1 may be 0.2 ≤ y1 ≤ 0.39, and z1 may be 0.01 ≤ z1 < 0.1.

A content (e.g., amount) of the positive electrode active material may be about 90 wt% to about 98 wt% based on the total weight of the positive electrode active material layer.

In some embodiments of the present disclosure, the positive electrode active material layer may include a binder. A content (e.g., amount) of the binder may be about 1 wt% to about 5 wt% based on the total weight of the positive electrode active material layer.

The binder improves binding properties of positive electrode active material particles with one another and with a positive electrode current collector. Examples thereof may be polyvinyl alcohol, carboxylmethyl cellulose, hydroxypropyl cellulose, diacetyl cellulose, polyvinylchloride, carboxylated polyvinylchloride, polyvinylfluoride, an ethylene oxide-containing polymer, polyvinylpyrrolidone, polyurethane, polytetrafluoroethylene, polyvinylidene fluoride, polyethylene, polypropylene, a styrene-butadiene rubber, an acrylated styrene-butadiene rubber, an epoxy resin, nylon, and/or the like, but are not limited thereto.

Al foil may be utilized as the positive electrode current collector, but is not limited thereto.

The negative electrode includes a negative electrode current collector and a negative electrode active material layer including a negative electrode active material formed on the negative electrode current collector.

The negative electrode active material may include a material that reversibly intercalates/deintercalates lithium ions, a lithium metal, a lithium metal alloy, a material capable of doping/dedoping lithium, or transition metal oxide.

The material that reversibly intercalates/deintercalates lithium ions may be a carbon material which is any generally-utilized carbon-based negative electrode active material in a rechargeable lithium battery and examples thereof may be crystalline carbon, amorphous carbon, or a combination thereof. Examples of the crystalline carbon may be graphite such as amorphous, sheet-shape, flake, spherical shape or fiber-shaped natural graphite or artificial graphite. Examples of the amorphous carbon may be soft carbon or hard carbon, a mesophase pitch carbonized product, calcined coke, and/or the like.

The lithium metal alloy may be an alloy of lithium and a metal selected from among Na, K, Rb, Cs, Fr, Be, Mg, Ca, Sr, Si, Sb, Pb, In, Zn, Ba, Ra, Ge, Al, and Sn.

The material capable of doping and dedoping lithium may be Si, a Si-C composite, SiOₓ (0 < x < 2), SiO₂, a Si-Q alloy (wherein Q is an element selected from an alkali metal, an alkaline-earth metal, a Group 13 element, a Group 14 element except for Si, a Group 15 element, a Group 16 element, a transition metal, a rare earth element, and a combination thereof), Sn, SnO_{y} (0 < y < 2), SnO₂, Sn-R¹¹ alloy (wherein R¹¹ is an element selected from an alkali metal, an alkaline-earth metal, a Group 13 element, a Group 14 element except for Sn, a Group 15 element, a Group 16 element, a transition metal, a rare earth element, and a combination thereof), and a combination thereof.

The elements Q and R¹¹ may be selected from among Mg, Ca, Sr, Ba, Ra, Sc, Y, Ti, Zr, Hf, Rf, V, Nb, Ta, Db, Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn (R¹¹ does not comprise Sn), In, Tl, Ge, P, As, Sb, Bi, S, Se, Te, Po, and a combination thereof.

The transition elements oxide may include vanadium oxide, lithium vanadium oxide, or lithium titanium oxide.

In some embodiments, the negative electrode active material may include at least one selected from among graphite and a Si composite.

The Si composite includes: a core including Si-based particles; and an amorphous carbon coating layer. For example, the Si-based particles may include at least one selected from among silicon particles, a Si-C composite, SiOₓ (0 < x ≤ 2), and an Si alloy.

For example, voids may be included in the central portion of the core including the Si-based particles, a radius of the central portion corresponds to 30% to 50% of a radius of the Si composite, an average particle diameter of the Si composite (particles) may be about 5 µm to about 20 µm, and an average particle diameter of the Si-based particles may be about 10 nm to about 200 nm.

In the present specification, the average particle diameter (D50) may be a particle size at 50% by volume in a cumulative size-distribution curve.

When the average particle diameter of the Si-based particles is within the above range, volume expansion occurring during charging and discharging may be suppressed or reduced, and a break in a conductive path due to particle crushing during charging and discharging may be prevented or reduced.

The core including the Si-based particles further includes amorphous carbon, and the central portion does not include amorphous carbon, and the amorphous carbon may exist only on the surface portion of the Si composite.

Here, the surface portion refers to a region from the outermost surface of the central portion to the outermost surface of the Si composite.

In some embodiments, the Si-based particles are substantially uniformly included throughout the Si composite, that is, may be present in a substantially uniform concentration in the central portion and surface portion.

The amorphous carbon may be soft carbon, hard carbon, a mesophase pitch carbonized product, calcined coke, or a combination thereof.

For example, the Si-C composite may include: silicon particles; and crystalline carbon.

The silicon particles may be included in an amount of about 1 to about 60 wt%, for example, about 3 to about 60 wt% based on the total weight of the Si-C composite.

The crystalline carbon may be, for example, graphite, and for example natural graphite, artificial graphite, or a combination thereof.

An average particle diameter of the crystalline carbon (particles) may be about 5 µm to about 30 µm.

When the negative electrode active material includes both (e.g., simultaneously) graphite and Si composite, the graphite and Si composite may be included in a mixture form, and in this case, the graphite and Si composite may be included in a weight ratio of about 99:1 to about 50:50.

For example, the graphite and Si composite may be included in a weight ratio of about 97:3 to about 80:20, or about 95:5 to about 80:20.

The amorphous carbon precursor may include a coal-based pitch, mesophase pitch, petroleum-based pitch, coal-based oil, petroleum-based heavy oil, or a polymer resin such as a phenol resin, a furan resin, or a polyimide resin.

In the negative electrode active material layer, the negative electrode active material may be included in an amount of about 95 wt% to about 99 wt% based on the total weight of the negative electrode active material layer.

In some embodiments of the present disclosure, the negative electrode active material layer further includes a binder, and optionally a conductive material. In the negative electrode active material layer, a content (e.g., amount) of the binder may be about 1 wt% to about 5 wt% based on the total weight of the negative electrode active material layer. When the negative electrode active material layer includes a conductive material, the negative electrode active material layer includes about 90 wt% to about 98 wt% of the negative electrode active material, about 1 wt% to about 5 wt% of the binder, and about 1 wt% to about 5 wt% of the conductive material.

The binder improves binding properties of negative electrode active material particles with one another and with a negative electrode current collector. The binder includes a non-water-soluble binder, a water-soluble binder, or a combination thereof.

The non-water-soluble binder may be selected from among polyvinylchloride, carboxylated polyvinylchloride, polyvinylfluoride, polyurethane, polytetrafluoroethylene, polyvinylidene fluoride, polyethylene, polypropylene, polyamideimide, polyimide, and a combination thereof.

The water-soluble binder may be a rubber-based binder or a polymer resin binder. The rubber-based binder may be selected from among a styrene-butadiene rubber, an acrylated styrene-butadiene rubber (SBR), an acrylonitrile-butadiene rubber, an acrylic rubber, a butyl rubber, a fluorine rubber, and a combination thereof. The polymer resin binder may be selected from among polytetrafluoroethylene, ethylenepropylen copolymer, polyethyleneoxide, polyvinylpyrrolidone, polyepichlorohydrine, polyphosphazene, polyacrylonitrile, polystyrene, an ethylenepropylenediene copolymer, polyvinylpyridine, chlorosulfonated polyethylene, latex, a polyester resin, an acrylic resin, a phenolic resin, an epoxy resin, polyvinyl alcohol, and a combination thereof.

When the water-soluble binder is utilized as a negative electrode binder in the negative electrode active material layer, a cellulose-based compound may be further utilized to provide viscosity as a thickener. The cellulose-based compound includes one or more of carboxymethyl cellulose, hydroxypropylmethyl cellulose, methyl cellulose, and alkali metal salts thereof. The alkali metals may be Na, K, or Li. Such a thickener may be included in an amount of about 0.1 parts by weight to about 3 parts by weight based on 100 parts by weight of the negative electrode active material.

The conductive material is included to provide electrode conductivity and any electrically conductive material may be utilized as a conductive material unless it causes a chemical change. Examples of the conductive material include a carbon-based material such as natural graphite, artificial graphite, carbon black, acetylene black, ketjen black, a carbon fiber, and/or the like; a metal-based material of a metal powder or a metal fiber including copper, nickel, aluminum silver, and/or the like; a conductive polymer such as a polyphenylene derivative; or a mixture thereof.

The negative electrode current collector may be selected from among a copper foil, a nickel foil, a stainless steel foil, a titanium foil, a nickel foam, a copper foam, a polymer substrate coated with a conductive metal, and a combination thereof.

A separator may exist between the positive electrode and the negative electrode depending on the type or kind of the rechargeable lithium battery. Such a separator may include polyethylene, polypropylene, polyvinylidene fluoride, or multilayers thereof such as a polyethylene/polypropylene double-layered separator, a polyethylene/polypropylene/polyethylene triple-layered separator, and a polypropylene/polyethylene/polypropylene triple-layered separator.

The rechargeable lithium battery may have a charging upper limit voltage greater than or equal to about 4.35 V. For example, the charging upper limit voltage may be in a range from about 4.35 V to about 4.55 V.

Hereinafter, examples of the present disclosure and comparative examples are described. These examples, however, are not in any sense to be interpreted as limiting the scope of the present disclosure.

### Manufacture of Rechargeable Lithium Battery Cell

### Example 1

LiNi_{0.75}Mn_{0.23}Al_{0.02}O₂ as a positive electrode active material, polyvinylidene fluoride as a binder, and acetylene black as a conductive material were mixed in a weight ratio of 96:3:1, respectively, and were dispersed in N-methyl pyrrolidone to prepare a positive electrode active material slurry.

The positive electrode active material slurry was coated on a 15 µm-thick Al foil, dried at 100 °C, and then pressed to prepare a positive electrode.

As a negative electrode active material, a mixture of artificial graphite and a Si composite in a weight ratio of 93:7 was utilized, and the negative electrode active material, a styrene-butadiene rubber binder, and carboxymethyl cellulose were mixed in a weight ratio of 98:1:1, respectively, and dispersed in distilled water to prepare a negative electrode active material slurry.

The Si composite included a core including artificial graphite and silicon particles, and a coal-based pitch is coated on the surface of the core.

The negative electrode active material slurry was coated on a 10 µm-thick Cu foil, dried at 100 °C, and then pressed to prepare a negative electrode.

An electrode assembly was prepared by assembling the prepared positive and negative electrodes and a polyethylene separator having a thickness of 25 µm, and an electrolyte was injected to prepare a rechargeable lithium battery cell.

A composition of the electrolyte is as follows.

### Composition of Electrolyte

Lithium salt: 1.5 M LiPF₆

Non-aqueous organic solvent: ethylmethyl carbonate: dimethyl carbonate (EMC:DMC= volume ratio of 20:80)

Additive: 0.5 parts by weight of the compound represented by Chemical Formula 1-1

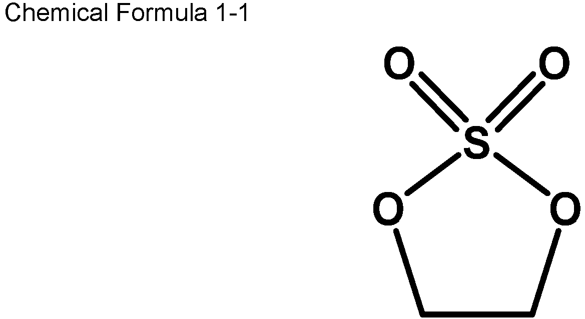

In the composition of the electrolyte, "parts by weight" refers to a relative weight of additives based on 100 parts by weight of the total electrolyte (lithium salt+non-aqueous organic solvent) excluding additives.

### Example 2

A rechargeable lithium battery cell was manufactured in substantially the same manner as in Example 1, except that 1 part by weight of the compound represented by Chemical Formula 1-1 was added.

### Example 3

A rechargeable lithium battery cell was manufactured in substantially the same manner as in Example 1, except that the compound represented by Chemical Formula 1-2 was added instead of the compound represented by Chemical Formula 1-1.

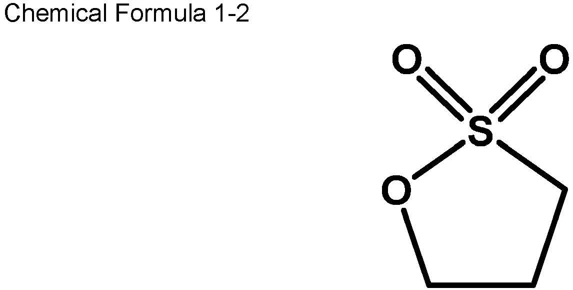

### Example 4

A rechargeable lithium battery cell was manufactured in substantially the same manner as in Example 1, except that 1 part by weight of the compound represented by Chemical Formula 1-2 was added.

### Comparative Example 1

A rechargeable lithium battery cell was manufactured in substantially the same manner as in Example 1, except that the compound represented by Chemical Formula 1-1 was not added to the composition of the electrolyte.

### Comparative Example 2

A rechargeable lithium battery cell was manufactured in substantially the same manner as in Example 1, except that 20 wt% of ethylene carbonate based on the total weight of the non-aqueous organic solvent in the composition of the electrolyte was added.

### Comparative Example 3

A rechargeable lithium battery cell was manufactured in substantially the same manner as in Example 3, except that 20 wt% of ethylene carbonate based on the total weight of the non-aqueous organic solvent in the composition of the electrolyte was added.

### Comparative Example 4

A rechargeable lithium battery cell was manufactured in substantially the same manner as in Example 1, except that 0.5 parts by weight of fluoroethylene carbonate (FEC) in the composition of the electrolyte was added instead of the compound represented by Chemical Formula 1-1.

### Examples 5 and 6

Each of rechargeable lithium battery cells was manufactured in substantially the same manner as in Examples 1 and 3, except that the non-aqueous organic solvent was changed into 100 volume% of dimethyl carbonate, i.e., only dimethyl carbonate was used as the non-aqueous organic solvent.

### Comparative Examples 5 to 10

Each of rechargeable lithium battery cells was manufactured in substantially the same manner as in Examples 1 to 4 and Comparative Examples 1 and 2, except that the positive electrode active material was changed into LiCoO₂.

### Comparative Examples 11 to 16

Each of rechargeable lithium battery cells was manufactured in substantially the same manner as in Examples 1 to 4 and Comparative Examples 1 and 2, except that the positive electrode active material was changed into LiNi_{0.5}Co_{0.2}Al_{0.3}O₂.

### Comparative Examples 17 to 22

Each of rechargeable lithium battery cells was manufactured in substantially the same manner as in Examples 1 to 4 and Comparative Examples 1 and 2, except that the positive electrode active material was changed into LiNi_{0.8}Co_{0.1}Mn_{0.1}O₂.

### Examples 7 and 8 and Comparative Example 23

Each of rechargeable lithium battery cells was manufactured in substantially the same manner as in Example 1, except that a mixing ratio of ethylmethyl carbonate and dimethyl carbonate was changed to a volume ratio of 30: 70 (Example 7), a volume ratio of 40: 60 (Example 8), and a volume ratio of 70: 30 (Comparative Example 23).

### Examples 9 and 10 and Comparative Example 24

Each of rechargeable lithium battery cells was manufactured in substantially the same manner as in Example 3, except that a mixing ratio of ethylmethyl carbonate and dimethyl carbonate was changed to a volume ratio of 30: 70 (Example 9), a volume ratio of 40: 60 (Example 10), and a volume ratio of 70: 30 (Comparative Example 24).

Each composition is shown in Table 1.

**Table 1**

| | Positive electrode active material | Composition of the electrolyte |
|---|---|---|
| Ex. 1 | LiNi_{0.75}Mn_{0.23}Al_{0.02}O₂ | 1.5M LiPF₆ in EMC/DMC 2/8 Chemical Formula 1-1 0.5 parts by weight |
| Ex. 2 | | 1.5M LiPF₆ in EMC/DMC 2/8 Chemical Formula 1-1 1 part by weight |
| Ex. 3 | | 1.5M LiPF₆ in EMC/DMC 2/8 Chemical Formula 1-2 0.5 parts by weight |
| Ex. 4 | | 1.5M LiPF₆ in EMC/DMC 2/8 Chemical Formula 1-2 1 part by weight |
| Ex. 5 | | 1.5M LiPF₆ in EMC/DMC 0/100 Chemical Formula 1-1 0.5 parts by weight |
| Ex. 6 | | 1.5M LiPF₆ in EMC/DMC 0/100 Chemical Formula 1-2 0.5 parts by weight |
| Ex. 7 | | 1.5M LiPF₆ in EMC/DMC 3/7 Chemical Formula 1-1 0.5 parts by weight |
| Ex. 8 | | 1.5M LiPF₆ in EMC/DMC 4/6 Chemical Formula 1-1 0.5 parts by weight |
| Ex. 9 | | 1.5M LiPF₆ in EMC/DMC 3/7 Chemical Formula 1-2 0.5 parts by weight |
| Ex. 10 | | 1.5M LiPF₆ in EMC/DMC 4/6 Chemical Formula 1-2 0.5 parts by weight |
| Comp. Ex. 1 | LiNi_{0.75}Mn_{0.23}Al_{0.02}O₂ | 1.5M LiPF₆ in EMC/DMC 2/8 |
| Comp. Ex. 2 | | 1.5M LiPF₆ in EMC/DMC 2/8 Chemical Formula 1-1 0.5 parts by weight |
| | | EC 20 wt% |
| Comp. Ex. 3 | | 1.5M LiPF₆ in EMC/DMC 2/8 Chemical Formula 1-2 0.5 parts by weight |
| | | EC 20 wt% |
| Comp. Ex. 4 | | 1.5M LiPF₆ in EMC/DMC 2/8 FEC 0.5 parts by weight EC 20 wt% |
| Comp. Ex. 5 | LiCoO₂ | 1.5M LiPF₆ in EMC/DMC 2/8 Chemical Formula 1-1 0.5 parts by weight |
| Comp. Ex. 6 | | 1.5M LiPF₆ in EMC/DMC 2/8 Chemical Formula 1-1 1 part by weight |
| Comp. Ex. 7 | | 1.5M LiPF₆ in EMC/DMC 2/8 Chemical Formula 1-2 0.5 parts by weight |
| Comp. Ex. 8 | | 1.5M LiPF₆ in EMC/DMC 2/8 Chemical Formula 1-2 1 part by weight |
| Comp. Ex. 9 | | 1.5M LiPF₆ in EMC/DMC 2/8 |
| Comp. Ex. 10 | | 1.5M LiPF₆ in EMC/DMC 2/8 Chemical Formula 1-1 0.5 parts by weight |
| | | EC 20 wt% |
| Comp. Ex. 11 | LiNi_{0.5}Co_{0.2}Al_{0.3}O₂ | 1.5M LiPF₆ in EMC/DMC 2/8 Chemical Formula 1-1 0.5 parts by weight |
| Comp. Ex. 12 | | 1.5M LiPF₆ in EMC/DMC 2/8 Chemical Formula 1-1 1 part by weight |
| Comp. Ex. 13 | | 1.5M LiPF₆ in EMC/DMC 2/8 Chemical Formula 1-2 0.5 parts by weight |
| Comp. Ex. 14 | | 1.5M LiPF₆ in EMC/DMC 2/8 Chemical Formula 1-2 1 part by weight |
| Comp. Ex. 15 | | 1.5M LiPF₆ in EMC/DMC 2/8 |
| Comp. Ex. 16 | | 1.5M LiPF₆ in EMC/DMC 2/8 Chemical Formula 1-1 0.5 parts by weight |
| | | EC 20 wt% |
| Comp. Ex. 17 | LiNi_{0.8}Co_{0.1}Mn_{0.1}O₂ | 1.5M LiPF₆ in EMC/DMC 2/8 Chemical Formula 1-1 0.5 parts by weight |
| Comp. Ex. 18 | | 1.5M LiPF₆ in EMC/DMC 2/8 Chemical Formula 1-1 1 part by weight |
| Comp. Ex. 19 | | 1.5M LiPF₆ in EMC/DMC 2/8 Chemical Formula 1-2 0.5 parts by weight |
| Comp. Ex. 20 | | 1.5M LiPF₆ in EMC/DMC 2/8 Chemical Formula 1-2 1 part by weight |
| Comp. Ex. 21 | | 1.5M LiPF₆ in EMC/DMC 2/8 |
| Comp. Ex. 22 | | 1.5M LiPF₆ in EMC/DMC 2/8 Chemical Formula 1-1 0.5 parts by weight |
| | | EC 20 wt% |
| Comp. Ex. 23 | LiNi_{0.75}Mn_{0.23}Al_{0.02}O₂ | 1.5M LiPF₆ in EMC/DMC 7/3 Chemical Formula 1-1 0.5 parts by weight |
| Comp. Ex. 24 | | 1.5M LiPF₆ in EMC/DMC 7/3 Chemical Formula 1-2 0.5 parts by weight |

### Evaluation 1: Evaluation of Storage Characteristics at High Temperature

The rechargeable lithium battery cells according to Examples 1 to 10 and Comparative Examples 1 to 24 were measured with respect to initial DC internal resistance (DCIR) as △V/△I (change in voltage/change in current), and after setting a maximum energy state inside the rechargeable lithium battery cells to a fully charged state (SOC 100%), the rechargeable lithium battery cells were stored at a high temperature (60 °C) for 30 days and then, measured again with respect to DC internal resistance to calculate a DCIR increase rate (%) according to Equation 1, and the results are shown in Tables 2, 3, and 6. DCIR increase rate = {(DCIR after 30 days storage at high temperature) / (initial DCIR)} X 100

### Evaluation 2: Evaluation of Cycle-life Characteristics at High Temperature

The rechargeable lithium battery cells according to Examples 1 to 6 and Comparative Examples 1 to 4 were once charged and discharged at 0.2 C and then, measured with respect to charge and discharge capacity.

In some embodiments, the rechargeable lithium battery cells according to Examples 1 to 6 and Comparative Examples 1 to 4 were charged to an upper limit voltage of 4.35 V to 4.45 V and discharged at 0.2 C to 2.5 V under a constant current condition to measure initial discharge capacity.

Then, the rechargeable lithium battery cells were 200 cycles charged at 0.33 C (CC/CV, 4.35 V and 4.45 V, 0.025 C cut-off) and discharged at 1.0 C (CC, 2.5 V cut-off) at 45 °C and then, measured with respect to discharge capacity.

A ratio of the discharge capacity to the initial discharge capacity was calculated and provided as a capacity recovery rate (%, recovery), and a capacity recovery increase rate relative to Comparative Example 2 was calculated according to Equation 2. The results are shown in Table 5. Capacity recovery increase rate relative to Comparative Example 2 = {(capacity recovery rate)/(capacity recovery rate of Comparative Example 2)} X 100

### Evaluation 3: Measurement of Gas Generation after High-temperature Storage

The rechargeable lithium battery cells according to Examples 1 to 6 and Comparative Examples 1 to 4 were left at 60 °C for 30 days and then, measured with respect to an amount of generated gas (mL) at the 10th day and the 30th day through a refinery gas-analysis (RGA) to calculate a gas generation decrease rate relative to Comparative Example 2 according Equation 3. The results are shown in Table 4. Gas generation decrease rate relative to Comparative Example 2 = {(Gas generation on the 30th day)-(Gas generation on the 30th day of Comparative Example 2)}/ (Gas generation on 30th day of Comparative Example 2) X 100

**Table 2**

| | Initial DCIR (mOhm) | DCIR (mOhm) after storage at high temperature (60 °C) for 30 days | DCIR Increase rate (60 °C, 30 days) (%) |
|---|---|---|---|
| Ex. 1 | 39.46 | 46.88 | 118.8 |
| Ex. 2 | 40.12 | 45.82 | 114.2 |
| Ex. 3 | 39.25 | 46.86 | 119.4 |
| Ex. 4 | 39.87 | 45.37 | 113.8 |
| Ex. 5 | 38.47 | 44.32 | 115.2 |
| Ex. 6 | 37.86 | 43.50 | 114.9 |
| Comp. Ex. 1 | 38.89 | 47.68 | 122.6 |
| Comp. Ex. 2 | 41.59 | 52.74 | 126.8 |
| Comp. Ex. 3 | 41.25 | 52.06 | 126.2 |
| Comp. Ex. 4 | 41.37 | 50.55 | 122.2 |

**Table 3**

| Positive electrode active material | | Initial DCIR (mOhm) | DCIR (mOhm) after storage at high temperature (60 °C) for 30 days | DCIR increase rate (60 °C, 30 days) (%) |
|---|---|---|---|---|
| LiNi_{0.75}Mn_{0.23}Al_{0.02}O₂ | Ex. 1 | 39.46 | 46.88 | 118.8 |
| | Ex. 2 | 40.12 | 45.82 | 114.2 |
| | Ex. 3 | 39.25 | 46.86 | 119.4 |
| | Ex. 4 | 39.87 | 45.37 | 113.8 |
| | Comp. Ex. 1 | 38.89 | 47.68 | 122.6 |
| | Comp. Ex. 2 | 41.59 | 52.74 | 126.8 |
| LiCoO₂ | Comp. Ex. 5 | 41.50 | 52.46 | 126.4 |
| | Comp. Ex. 6 | 41.63 | 51.33 | 123.3 |
| | Comp. Ex. 7 | 41.53 | 52.16 | 125.6 |
| | Comp. Ex. 8 | 41.61 | 51.22 | 123.1 |
| | Comp. Ex. 9 | 41.42 | 53.72 | 129.7 |
| | Comp. Ex. 10 | 40.05 | 49.50 | 123.6 |
| LiNi_{0.5}Co_{0.2}Al_{0.3}O₂ | Comp. Ex. 11 | 42.23 | 54.18 | 128.3 |
| | Comp. Ex. 12 | 42.36 | 51.98 | 122.7 |
| | Comp. Ex. 13 | 42.25 | 54.00 | 127.8 |
| | Comp. Ex. 14 | 42.38 | 51.96 | 122.6 |
| | Comp. Ex. 15 | 42.14 | 55.25 | 131.1 |
| | Comp. Ex. 16 | 40.37 | 49.98 | 123.8 |
| LiNi_{0.8}Cp_{0.1}Mn_{0.1}O₂ | Comp. Ex. 17 | 41.37 | 52.33 | 126.5 |
| | Comp. Ex. 18 | 41.55 | 51.15 | 123.1 |
| | Comp. Ex. 19 | 41.48 | 52.06 | 125.5 |
| | Comp. Ex. 20 | 41.61 | 51.01 | 122.6 |
| | Comp. Ex. 21 | 41.36 | 53.64 | 129.7 |
| | Comp. Ex. 22 | 39.95 | 49.22 | 123.2 |

**Table 4**

| | Amount of gas generated after storage at high temperature (60 °C) | | |
|---|---|---|---|
| | 10th day (mL) | 30th day (mL) | Gas generation decrease rate (%) relative to Comparative Example 2 |
| Ex. 1 | 12.96 | 34.21 | 7.99 |
| Ex. 2 | 12.39 | 32.37 | 12.94 |
| Ex. 3 | 13.16 | 34.71 | 6.64 |
| Ex. 4 | 12.29 | 32.24 | 13.29 |
| Ex. 5 | 13.01 | 32.61 | 12.29 |
| Ex. 6 | 13.22 | 32.93 | 11.43 |
| Comp. Ex. 1 | 13.76 | 36.59 | 1.59 |
| Comp. Ex. 2 | 13.64 | 37.18 | 0.00 |
| Comp. Ex. 3 | 13.49 | 36.62 | 1.51 |
| Comp. Ex. 4 | 14.52 | 36.26 | 2.47 |

**Table 5**

| | | Capacity recovery rate (45 °C, 200th cy, %) | Capacity recovery increase rate relative to Comparative Example 2 (%) |
|---|---|---|---|
| 4.45 V | Ex. 1 | 88.6 | 107.0 |
| | Ex. 2 | 93.1 | 112.4 |
| | Ex. 3 | 88.20 | 106.5 |
| | Ex. 4 | 92.30 | 111.5 |
| | Ex. 5 | 91.42 | 110.4 |
| | Ex. 6 | 91.39 | 110.4 |
| | Comp. Ex. 1 | 85.6 | 103.4 |
| | Comp. Ex. 2 | 82.8 | 100.0 |
| | Comp. Ex. 3 | 82.4 | 99.5 |
| | Comp. Ex. 4 | 84.5 | 102.1 |
| 4.35 V | Ex. 1 | 89.50 | 105.8 |
| | Ex. 2 | 93.60 | 110.6 |
| | Ex. 3 | 90.60 | 107.1 |
| | Ex. 4 | 93.40 | 110.4 |
| | Ex. 5 | 94.12 | 111.3 |
| | Ex. 6 | 94.87 | 112.1 |
| | Comp. Ex. 1 | 87.8 | 103.8 |
| | Comp. Ex. 2 | 84.6 | 100.0 |
| | Comp. Ex. 3 | 84.4 | 99.8 |
| | Comp. Ex. 4 | 85.7 | 101.3 |

**Table 6**

| | Initial DCIR (mOhm) | DCIR (mOhm) after storage at high temperature (60 °C) for 30 days | DCIR increase rate (60° C, 30 days) (%) |
|---|---|---|---|
| Ex. 1 (EMC:DMC=20:80) | 39.46 | 46.88 | 118.8 |
| Ex. 7 (EMC:DMC=30:70) | 40.09 | 47.83 | 119.3 |
| Ex. 8 (EMC:DMC=40:60) | 40.83 | 49.40 | 121.0 |
| Comp. Ex. 2 EC 20wt% | 41.59 | 52.74 | 126.8 |
| Comp. Ex. 23 (EMC:DMC=70:30) | 41.22 | 50.41 | 122.3 |
| Ex. 3 (EMC:DMC=20:80) | 39.25 | 46.86 | 119.4 |
| Ex. 9 (EMC:DMC=30:70) | 39.88 | 47.98 | 120.3 |
| Ex. 10 (EMC:DMC=40:60) | 40.52 | 49.07 | 121.1 |
| Comp. Ex. 3 EC 20wt% | 41.25 | 52.06 | 126.2 |
| Comp. Ex. 24 (EMC:DMC=70:30) | 41.46 | 50.50 | 121.8 |

Referring to Tables 2 to 6, DCIR increase rates were decreased by the combinations of an electrolyte and a cobalt-free positive electrode active material according to the present disclosure, improving high-temperature storage characteristics and high-temperature charge and discharge characteristics.

In some embodiments, the rechargeable lithium battery cells according to the examples exhibited a significantly reduced amount of gas generated after stored at a high temperature.

In the present disclosure, if (e.g., when) particles are spherical, "size" or "particle diameter" indicates a particle diameter or an average particle diameter, and if (e.g., when) the particles are non-spherical, the "size" or "particle diameter" indicates a major axis length or an average major axis length. That is, if (e.g., when) particles are spherical, "particle diameter" indicates a particle diameter, and when the particles are non-spherical, the "particle diameter" indicates a major axis length. The size or diameter of the particles may be measured utilizing a scanning electron microscope or a particle size analyzer. As the particle size analyzer, for example, HORIBA, LA-950 laser particle size analyzer, may be utilized. When the size of the particles is measured utilizing a particle size analyzer, the average particle diameter (or size) is referred to as D50. D50 refers to the average diameter (or size) of particles whose cumulative volume corresponds to 50 vol% in the particle size distribution (e.g., cumulative distribution), and refers to the value of the particle size corresponding to 50% from the smallest particle when the total number of particles is 100% in the distribution curve accumulated in the order of the smallest particle size to the largest particle size.

### Description of symbols

100: rechargeable lithium battery
112: negative electrode
113: separator
114: positive electrode
120: battery case
140: sealing member

## Claims

1. A rechargeable lithium battery (100) comprising:
an electrolyte comprising a non-aqueous organic solvent, a lithium salt, and an additive;
a positive electrode (114) comprising a positive electrode active material; and
a negative electrode (112) comprising a negative electrode active material,
wherein the non-aqueous organic solvent is only composed of a chain carbonate, the positive electrode active material comprises a cobalt-free lithium nickel manganese-based oxide,
wherein the positive electrode active material is represented by Chemical Formula 4:
Chemical Formula 4 LiₐNiₓMn_{y}M¹_{z}M²_{w}O_{2±b}X_{c}, and
wherein, in Chemical Formula 4, 0.5 ≤ a < 1.8, 0 ≤ b ≤ 0.1, 0 ≤ c ≤ 0.1, 0 ≤ w < 0.1, 0.6 ≤ x < 1.0, 0 < y < 0.4, 0 < z < 0.1, w + x + y + z = 1, M¹ and M² are each independently one or more elements selected from among Al, Mg, Ti, Zr, Sr, V, B, W, Mo, Si, Ba, Ca, Ce, Cr, Fe, and Nb, and X is one or more elements selected from among S, F, P, and Cl, and
the additive comprises a compound represented by Chemical Formula 1: 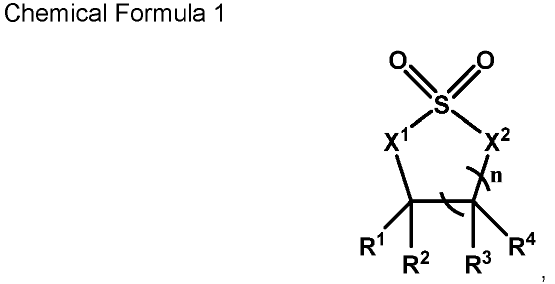 and
wherein, in Chemical Formula 1,
X¹ and X² are each independently O, S, or CR^{a}R^{b},
at least one selected from among X¹ and X² is O, R^{a}, R^{b}, and R¹ to R⁴ are each independently hydrogen, a halogen, a substituted or
unsubstituted C1 to C20 alkyl group, a substituted or unsubstituted C1 to C20 alkoxy group, a substituted or unsubstituted C2 to C20 alkenyl group, a substituted or
unsubstituted C3 to C20 alkynyl group, a substituted or unsubstituted C3 to C20 cycloalkyl group, a substituted or unsubstituted C6 to C50 aryl group, a substituted or
unsubstituted C7 to C50 alkylaryl group, or a substituted or unsubstituted C6 to C50 heteroaryl group, and
n is an integer of 1 or 2,
wherein "substituted" refers to replacement of at least one selected from among hydrogen of a substituent or a compound by deuterium, a halogen, a hydroxyl group,
an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group,
a C2 to C30 heteroaryl group, a C1 to C20 alkoxy group, a C1 to C10 fluoroalkyl group, and a cyano group.

2. The rechargeable lithium battery of claim 1, wherein
the chain carbonate is represented by Chemical Formula 2: 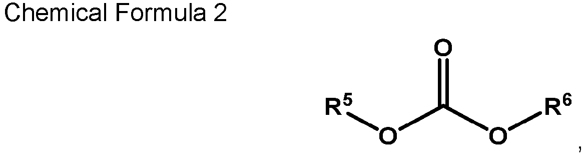 and
wherein, in Chemical Formula 2
R⁵ and R⁶ are each independently a substituted or unsubstituted C1 to C20 alkyl group,
wherein "substituted" refers to replacement of at least one selected from among hydrogen of a substituent or a compound by deuterium, a halogen, a hydroxyl group, an amino group, a C1 to C30 amine group, a nitro group, a C1 to C40 silyl group, a C1 to C30 alkyl group, a C1 to C10 alkylsilyl group, a C6 to C30 arylsilyl group, a C3 to C30 cycloalkyl group, a C3 to C30 heterocycloalkyl group, a C6 to C30 aryl group, a C2 to C30 heteroaryl group, a C1 to C20 alkoxy group, a C1 to C10 fluoroalkyl group, and a cyano group.

3. The rechargeable lithium battery of claim 2, wherein
the non-aqueous organic solvent comprises mixed solvent of at least two solvents selected from among dimethyl carbonate (DMC), diethyl carbonate (DEC), dipropyl carbonate (DPC), methylpropyl carbonate (MPC), ethylpropyl carbonate (EPC), and ethylmethyl carbonate (EMC).

4. The rechargeable lithium battery of claim 2 or 3, wherein
the non-aqueous organic solvent is dimethyl carbonate (DMC) or a combination of ethylmethyl carbonate (EMC) and dimethyl carbonate (DMC).

5. The rechargeable lithium battery of any one of the preceding claims, wherein Chemical Formula 1 is represented by Chemical Formula 1A or Chemical Formula 1B: 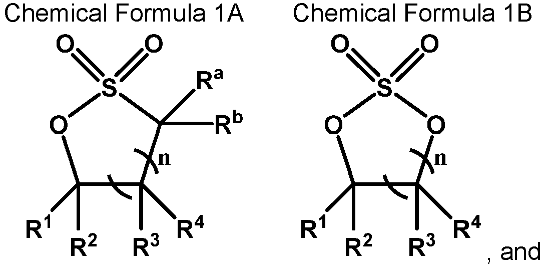
wherein, in Chemical Formula 1A and Chemical Formula 1B,
R^{a}, R^{b}, R¹ to R⁴, and n are the same as defined in Chemical Formula 1.

6. The rechargeable lithium battery of any one of the preceding claims, wherein R^{a}, R^{b}, and R¹ to R⁴ in Chemical Formula 1 are each independently hydrogen, a substituted or unsubstituted C1 to C10 alkyl group, or a substituted or unsubstituted C6 to C20 aryl group, wherein the term "substituted" is defined in the same way as in Chemical Formula 1.

7. The rechargeable lithium battery of any one of the preceding claims, wherein n in Chemical Formula 1 is 1.

8. The rechargeable lithium battery of claim 1, wherein
the compound represented by Chemical Formula 1 is selected from among the compounds listed in Group 1:

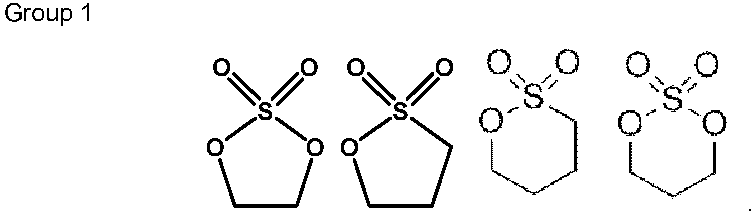

9. The rechargeable lithium battery of any one of the preceding claims, wherein the compound represented by Chemical Formula 1 is in an amount of 0.05 to 5.0 parts by weight based on 100 parts by weight of the total electrolyte excluding additives.

10. The rechargeable lithium battery of any one of the preceding claims, wherein the electrolyte further comprises at least one selected from among other additives of vinylene carbonate (VC), fluoroethylene carbonate (FEC), difluoroethylene carbonate, chloroethylene carbonate, dichloroethylene carbonate, bromoethylene carbonate, dibromoethylene carbonate, nitroethylene carbonate, cyanoethylene carbonate, vinylethylene carbonate (VEC), adiponitrile (AN), succinonitrile (SN), 1,3,6-hexane tricyanide (HTCN), propene sultone (PST), propane sultone (PS), lithium tetrafluoroborate (LiBF₄), lithium difluorophosphate (LiPO₂F₂), and 2-fluoro biphenyl (2-FBP).

11. The rechargeable lithium battery of claim 1, wherein
Chemical Formula 4 is represented by Chemical Formula 4-1:
Chemical Formula 4-1 LiₐNiₓ₁Mn_{y1}Al_{z1}M²_{w1}O_{2±b}X_{c} and
wherein, in Chemical Formula 4-1,
0.5 ≤ a < 1.8, 0 ≤ b ≤ 0.1, 0 ≤ c ≤ 0.1, 0 ≤ w1 < 0.1, 0.6 ≤ x1 < 1.0, 0 < y1 < 0.4, 0 < z1 < 0.1, w1 + x1 + y1 + z1 = 1,
M² is one or more elements selected from among Mg, Ti, Zr, Cr, Sr, V, B, W, Mo, Nb, Si, Ba, Ca, Ce, and Fe, and
X is one or more elements selected from among S, F, P, and Cl.

12. The rechargeable lithium battery of claim 11, wherein
in Chemical Formula 4-1, x1 is 0.6 ≤ x1 ≤ 0.79, y1 is 0.2 ≤ y1 ≤ 0.39, and z1 is 0.01 ≤ z1 < 0.1.

13. The rechargeable lithium battery of any one of the preceding claims, wherein the negative electrode active material comprises at least one selected from among graphite and a Si composite.

## Patentansprüche

1. Wiederaufladbare Lithiumbatterie (100), umfassend:
einen Elektrolyten, der ein nichtwässriges organisches Lösungsmittel, ein Lithiumsalz und
ein Additiv umfasst;
eine positive Elektrode (114), die ein Positivelektroden-Aktivmaterial umfasst; und
eine negative Elektrode (112), die ein Negativelektroden-Aktivmaterial umfasst,
wobei das nichtwässrige organische Lösungsmittel nur aus einem Kettencarbonat zusammengesetzt ist und das Positivelektroden-Aktivmaterial ein cobaltfreies Oxid auf Lithium-Nickel-Mangan-Basis umfasst,
wobei das Positivelektroden-Aktivmaterial durch die chemische Formel 4 dargestellt wird:
Chemische Formel 4 LiₐNiₓMn_{y}M¹_{z}M²_{w}O_{2±b}X_{c}, und
wobei, in der chemischen Formel 4, 0,5 ≤ a < 1,8, 0 ≤ b ≤ 0,1, 0 ≤ c ≤ 0,1, 0 ≤ w < 0,1, 0,6 ≤ x < 1,0, 0 < y < 0,4, 0 < z < 0,1, w + x + y + z = 1, M¹ und M² jeweils unabhängig ein oder mehrere Elemente sind, ausgewählt aus Al, Mg, Ti, Zr, Sr, V, B, W, Mo, Si, Ba, Ca, Ce, Cr, Fe und Nb, und X ein oder mehrere Elemente ist, ausgewählt aus S, F, P und Cl, und das Additiv eine durch die chemische Formel 1 dargestellte Verbindung umfasst: 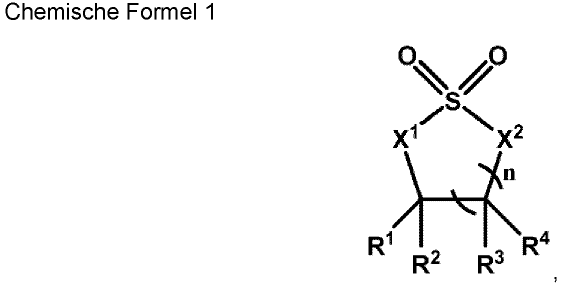 und
wobei, in der chemischen Formel 1,
X¹ und X² jeweils unabhängig O, S oder CR^{a}R^{b} sind,
mindestens eines, ausgewählt aus X¹ und X², O ist,
R^{a}, R^{b} und R¹ bis R⁴ jeweils unabhängig Wasserstoff, ein Halogen, ein substituierter oder unsubstituierter C₁- bis C₂₀-Alkylrest, ein substituierter oder unsubstituierter C₁- bis C₂₀-Alkoxyrest, ein substituierter oder unsubstituierter C₂- bis C₂₀-Alkenylrest, ein substituierter oder unsubstituierter C₃- bis C₂₀-Alkinylrest, ein substituierter oder unsubstituierter C₃- bis C₂₀-Cycloalkylrest, ein substituierter oder unsubstituierter C₆- bis C₅₀-Arylrest, ein substituierter oder unsubstituierter C₇- bis C₅₀-Alkylarylrest oder ein substituierter oder unsubstituierter C₆- bis C₅₀-Heteroarylrest sind, und
n eine ganze Zahl von 1 oder 2 ist,
wobei sich "substituiert" auf das Ersetzen von mindestens einem, ausgewählt aus Wasserstoff eines Substituenten oder einer Verbindung, durch Deuterium, ein Halogen, eine Hydroxylgruppe, eine Aminogruppe, einen C₁- bis C₃₀-Aminrest, eine Nitrogruppe, einen C₁- bis C₄₀-Silylrest, einen C₁- bis C₃₀-Alkylrest, einen C₁- bis C₁₀-Alkylsilylrest, einen C₆- bis C₃₀-Arylsilylrest, einen C₃- bis C₃₀-Cycloalkylrest, einen C₃- bis C₃₀-Heterocycloalkylrest, einen C₆- bis C₃₀-Arylrest, einen C₂- bis C₃₀-Heteroarylrest, einen C₁- bis C₂₀-Alkoxyrest, einen C₁- bis C₁₀-Fluoralkylrest und eine Cyanogruppe bezieht.

2. Wiederaufladbare Lithiumbatterie nach Anspruch 1, wobei
das Kettencarbonat durch die chemische Formel 2 dargestellt wird: 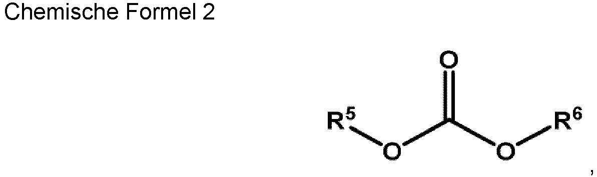 und
wobei, in der chemischen Formel 2,
R⁵ und R⁶ jeweils unabhängig ein substituierter oder unsubstituierter C₁- bis C₂₀-Alkylrest sind,
wobei sich "substituiert" auf das Ersetzen von mindestens einem, ausgewählt aus Wasserstoff eines Substituenten oder einer Verbindung, durch Deuterium, ein Halogen, eine Hydroxylgruppe, eine Aminogruppe, einen C₁- bis C₃₀-Aminrest, eine Nitrogruppe, einen C₁- bis C₄₀-Silylrest, einen C₁- bis C₃₀-Alkylrest, einen C₁- bis C₁₀-Alkylsilylrest, einen C₆- bis C₃₀-Arylsilylrest, einen C₃- bis C₃₀-Cycloalkylrest, einen C₃- bis C₃₀-Heterocycloalkylrest, einen C₆- bis C₃₀-Arylrest, einen C₂- bis C₃₀-Heteroarylrest, einen C₁- bis C₂₀-Alkoxyrest, einen C₁- bis C₁₀-Fluoralkylrest und eine Cyanogruppe bezieht.

3. Wiederaufladbare Lithiumbatterie nach Anspruch 2, wobei
das nichtwässrige organische Lösungsmittel ein Lösungsmittelgemisch aus mindestens zwei Lösungsmitteln, ausgewählt aus Dimethylcarbonat (DMC), Diethylcarbonat (DEC), Dipropylcarbonat (DPC), Methylpropylcarbonat (MPC), Ethylpropylcarbonat (EPC) und Ethylmethylcarbonat (EMC), umfasst.

4. Wiederaufladbare Lithiumbatterie nach Anspruch 2 oder 3, wobei
das nichtwässrige organische Lösungsmittel Dimethylcarbonat (DMC) oder eine Kombination von Ethylmethylcarbonat (EMC) und Dimethylcarbonat (DMC) ist.

5. Wiederaufladbare Lithiumbatterie nach einem der vorangehenden Ansprüche, wobei die chemische Formel 1 durch die chemische Formel 1A oder die chemische Formel 1B dargestellt wird: 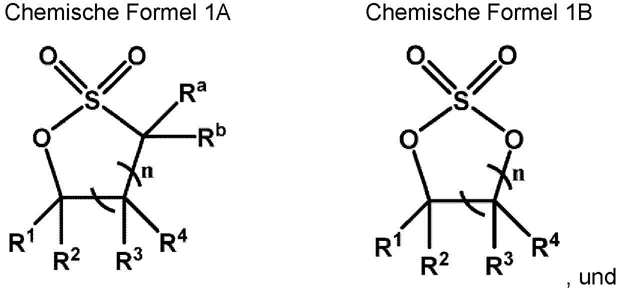 wobei, in der chemischen Formel 1A und der chemischen Formel 1B, R^{a}, R^{b}, R¹ bis R⁴ und n das Gleiche sind wie bei der chemischen Formel 1 definiert.

6. Wiederaufladbare Lithiumbatterie nach einem der vorangehenden Ansprüche, wobei R^{a}, R^{b} und R¹ bis R⁴ in der chemischen Formel 1 jeweils unabhängig Wasserstoff, ein substituierter oder unsubstituierter C₁- bis C₁₀-Alkylrest oder ein substituierter oder unsubstituierter C₆- bis C₂₀-Arylrest sind, wobei der Begriff "substituiert" auf die gleiche Weise wie bei der chemischen Formel 1 definiert ist.

7. Wiederaufladbare Lithiumbatterie nach einem der vorangehenden Ansprüche, wobei n in der chemischen Formel 1 1 ist.

8. Wiederaufladbare Lithiumbatterie nach Anspruch 1, wobei
die durch die chemische Formel 1 dargestellte Verbindung aus den in Gruppe 1 aufgeführten Verbindungen ausgewählt ist:

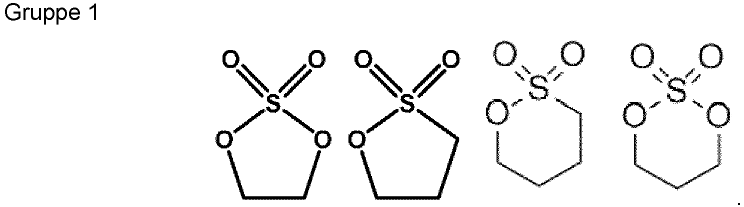

9. Wiederaufladbare Lithiumbatterie nach einem der vorangehenden Ansprüche, wobei die durch die chemische Formel 1 dargestellte Verbindung in einer Menge von 0,05 bis 5,0 Gewichtsteilen, bezogen auf 100 Gewichtsteile des gesamten Elektrolyten ohne Additive, vorliegt.

10. Wiederaufladbare Lithiumbatterie nach einem der vorangehenden Ansprüche, wobei der Elektrolyt ferner mindestens eines, ausgewählt aus den weiteren Additiven Vinylencarbonat (VC), Fluorethylencarbonat (FEC), Difluorethylencarbonat, Chlorethylencarbonat, Dichlorethylencarbonat, Bromethylencarbonat, Dibromethylencarbonat, Nitroethylencarbonat, Cyanoethylencarbonat, Vinylethylencarbonat (VEC), Adiponitril (AN), Succinonitril (SN), 1,3,6-Hexantricyanid (HTCN), Propensulton (PST), Propansulton (PS), Lithiumtetrafluoroborat (LiBF₄), Lithiumdifluorphosphat (LiPO₂F₂) und 2-Fluorbiphenyl (2-FBP), umfasst.

11. Wiederaufladbare Lithiumbatterie nach Anspruch 1, wobei
die chemische Formel 4 durch die chemische Formel 4-1 dargestellt wird:
Chemische Formel 4-1 LiₐNiₓ₁Mn_{y1}Al_{z1}M²_{w1}O_{2±b}X_{c}, und
wobei, in der chemischen Formel 4-1,
0,5 ≤ a < 1,8, 0 ≤ b ≤ 0,1, 0 ≤ c ≤ 0,1, 0 ≤ w1 < 0,1, 0,6 ≤ x1 < 1,0, 0 < y1 < 0,4, 0 < z1 < 0,1, w1 + x1 + y1 + z1 = 1,
M² ein oder mehrere Elemente ist, ausgewählt aus Mg, Ti, Zr, Cr, Sr, V, B, W, Mo, Nb, Si, Ba, Ca, Ce und Fe, und
X ein oder mehrere Elemente ist, ausgewählt aus S, F, P und Cl.

12. Wiederaufladbare Lithiumbatterie nach Anspruch 11, wobei
in der chemischen Formel 4-1 für x1 gilt: 0,6 ≤ x1 ≤ 0,79, für y1 gilt: 0,2 ≤ y1 ≤ 0,39 und für z1 gilt: 0,01 ≤ z1 < 0,1.

13. Wiederaufladbare Lithiumbatterie nach einem der vorangehenden Ansprüche, wobei das Negativelektroden-Aktivmaterial mindestens eines, ausgewählt aus Graphit und einem Si-Verbundstoff, umfasst.

## Revendications

1. Batterie au lithium rechargeable (100) comprenant :
un électrolyte comprenant un solvant organique non aqueux, un sel de lithium, et un additif ;
une électrode positive (114) comprenant un matériau actif d'électrode positive ; et
une électrode négative (112) comprenant un matériau actif d'électrode négative,
dans laquelle le solvant organique non aqueux est uniquement composé d'un carbonate linéaire,
le matériau actif d'électrode positive comprend un oxyde à base de lithium-nickel-manganèse sans cobalt,
dans laquelle le matériau actif d'électrode positive est représenté par la Formule Chimique 4 :
Formule Chimique 4 LiₐNiₓMn_{y}M¹_{z}M²_{w}O_{2±b}X_{c}, et
dans laquelle, dans la Formule Chimique 4, 0,5 ≤ a < 1,8, 0 ≤ b ≤ 0,1, 0 ≤ c ≤ 0,1, 0 ≤ w < 0,1, 0,6 ≤ x < 1,0, 0 < y < 0,4, 0 < z < 0,1, w + x + y + z = 1, M¹ et M² sont chacun indépendamment un ou plusieurs éléments choisis parmi Al, Mg, Ti, Zr, Sr, V, B, W, Mo, Si, Ba, Ca, Ce, Cr, Fe, et Nb, et X est un ou plusieurs éléments choisis parmi S, F, P, et Cl, et
l'additif comprend un composé représenté par la Formule Chimique 1 : 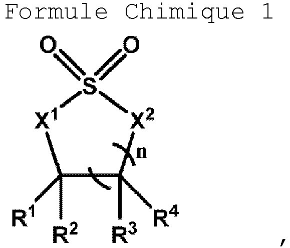 et
dans laquelle, dans la Formule Chimique 1,
X¹ et X² sont chacun indépendamment O, S, ou CR^{a}R^{b},
au moins un élément choisi parmi X¹ et X² est O,
R^{a}, R^{b} et R¹ à R⁴ sont chacun indépendamment un hydrogène, un halogène, un groupe alkyle en C1 à C20 substitué ou non substitué, un groupe alcoxy en C1 à C20 substitué ou non substitué, un groupe alcényle en C2 à C20 substitué ou non substitué, un groupe alcynyle en C3 à C20 substitué ou non substitué, un groupe cycloalkyle en C3 à C20 substitué ou non substitué, un groupe aryle C6 à C50 substitué ou non substitué, un groupe alkylaryle en C7 à C50 substitué ou non substitué, ou un groupe hétéroaryle en C6 à C50 substitué ou non substitué, et
n est un nombre entier de 1 ou 2,
dans laquelle « substitué » fait référence à un remplacement d'au moins un élément choisi parmi un hydrogène d'un substituant ou d'un composé par du deutérium, un halogène, un groupe hydroxyle, un groupe amino, un groupe amine en C1 à C30, un groupe nitro, un groupe silyle en C1 à C40, un groupe alkyle en C1 à C30, un groupe alkylsilyle en C1 à C10, un groupe arylsilyle en C6 à C30, un groupe cycloalkyle en C3 à C30, un groupe hétérocycloalkyle en C3 à C30, un groupe aryle en C6 à C30, un groupe hétéroaryle en C2 à C30, un groupe alcoxy en C1 à C20, un groupe fluoroalkyle en C1 à C10, et un groupe cyano.

2. Batterie au lithium rechargeable selon la revendication 1, dans laquelle
le carbonate linéaire est représenté par la Formule Chimique 2 : 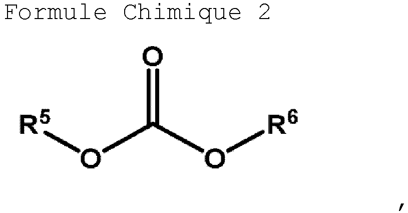 et
dans laquelle, dans la Formule Chimique 2
R⁵ et R⁶ sont chacun indépendamment un groupe alkyle en C1 à C20 substitué ou non substitué,
dans laquelle « substitué » fait référence à un remplacement d'au moins un élément choisi parmi un hydrogène d'un substituant ou d'un composé par du deutérium, un halogène, un groupe hydroxyle, un groupe amino, un groupe amine en C1 à C30, un groupe nitro, un groupe silyle en C1 à C40, un groupe alkyle en C1 à C30, un groupe alkylsilyle en C1 à C10, un groupe arylsilyle en C6 à C30, un groupe cycloalkyle en C3 à C30, un groupe hétérocycloalkyle en C3 à C30, un groupe aryle en C6 à C30, un groupe hétéroaryle en C2 à C30, un groupe alcoxy en C1 à C20, un groupe fluoroalkyle en C1 à C10, et un groupe cyano.

3. Batterie au lithium rechargeable selon la revendication 2, dans laquelle
le solvant organique non aqueux comprend un solvant mixte constitué d'au moins deux solvants choisis parmi du carbonate de diméthyle (DMC), carbonate de diéthyle (DEC), carbonate de dipropyle (DPC), carbonate de méthylpropyle (MPC), carbonate d'éthylpropyle (EPC), et carbonate d'éthylméthyle (EMC).

4. Batterie au lithium rechargeable selon la revendication 2 ou 3, dans laquelle
le solvant organique non aqueux est du carbonate de diméthyle (DMC) ou une combinaison de carbonate d'éthylméthyle (EMC) et de carbonate de diméthyle (DMC).

5. Batterie au lithium rechargeable selon l'une quelconque des revendications précédentes, dans laquelle la Formule Chimique 1 est représentée par la Formule Chimique 1A ou la Formule Chimique 1B : 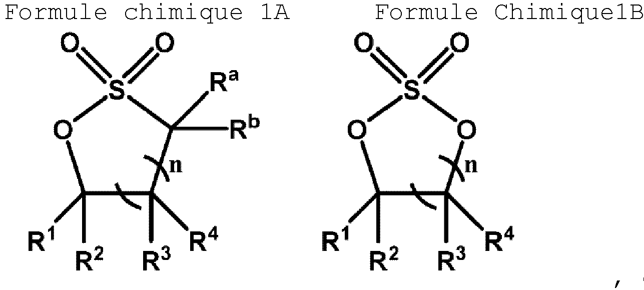 et
dans laquelle, dans la Formule Chimique 1A et la Formule Chimique 1B,
R^{a}, R^{b}, R¹ à R⁴, et n sont les mêmes que ceux définis dans la Formule Chimique 1.

6. Batterie au lithium rechargeable selon l'une quelconque des revendications précédentes, dans laquelle
R^{a}, R^{b}, et R¹ à R⁴ dans la Formule Chimique 1 sont chacun indépendamment un hydrogène, un groupe alkyle en C1 à C10 substitué ou non substitué, ou un groupe aryle en C6 à C20 substitué ou non substitué, le terme « substitué » étant défini de la même manière que dans la Formule Chimique 1.

7. Batterie au lithium rechargeable selon l'une quelconque des revendications précédentes, dans laquelle n dans la Formule Chimique 1 est 1.

8. Batterie au lithium rechargeable selon la revendication 1, dans laquelle
le composé représenté par la Formule Chimique 1 est choisi parmi les composés énumérés dans le Groupe 1 :

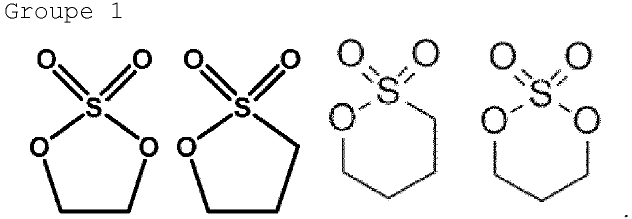

9. Batterie au lithium rechargeable selon l'une quelconque des revendications précédentes, dans laquelle
le composé représenté par la Formule Chimique 1 est présent selon une quantité de 0,05 à 5,0 parties en poids sur la base de 100 parties en poids de l'électrolyte total, à l'exclusion d'additifs.

10. Batterie au lithium rechargeable selon l'une quelconque des revendications précédentes, dans laquelle l'électrolyte comprend en outre au moins un additif choisi parmi d'autres additifs de carbonate de vinylène (VC), carbonate de fluoroéthylène (FEC), carbonate de difluoroéthylène, carbonate de chloroéthylène, carbonate de dichloroéthylène, carbonate de bromoéthylène, carbonate de dibromoéthylène, carbonate de nitroéthylène, carbonate de cyanoéthylène, carbonate de vinyléthylène (VEC), adiponitrile (AN), succinonitrile (SN), tricyanure de 1,3,6-hexane (HTCN), propène sultone (PST), propane sultone (PS), tétrafluoroborate de lithium (LiBF₄), difluorophosphate de lithium (LiPO₂F₂), et 2-fluoro biphényle (2-FBP).

11. Batterie au lithium rechargeable selon la revendication 1, dans laquelle
la Formule Chimique 4 est représentée par la Formule Chimique 4-1 :
Formule chimique 4-1 LiₐNiₓ₁Mn_{y1}Al_{z1}M²_{w1}O_{2±b}X_{c} et
dans laquelle, dans la Formule Chimique 4-1,
0,5 ≤ a < 1,8, 0 ≤ b ≤ 0,1, 0 ≤ c ≤ 0,1, 0 ≤ w1 < 0,1, 0,6 ≤ x1 < 1,0, 0 < y1 < 0,4, 0 < z1 < 0,1, w1 + x1 + y1 + z1 = 1,
M² est un ou plusieurs éléments choisis parmi Mg, Ti, Zr, Cr, Sr, V, B, W, Mo, Nb, Si, Ba, Ca, Ce, et Fe, et
X est un ou plusieurs éléments choisis parmi S, F, P, et Cl.

12. Batterie au lithium rechargeable selon la revendication 11, dans laquelle
dans la Formule Chimique 4-1, x1 est 0,6 ≤ x1 ≤ 0,79, y1 est 0,2 ≤ y1 ≤ 0,39, et z1 est 0,01 ≤ z1 < 0,1.

13. Batterie au lithium rechargeable selon l'une quelconque des revendications précédentes, dans laquelle le matériau actif d'électrode négative comprend au moins un élément choisi parmi du graphite et un composite de Si.
